# EUROPEAN PATENT APPLICATION

(11) **EP 0 613 675 A1**
(43) Date of publication of application: **07.09.1994**
(21) Application number: 94301605.5
(22) Date of filing: 04.03.1994
(51) Int. Cl.: A61K 7/00, A61K 9/70, A61K 31/44

(54) **Cosmetic applicator useful for cleansing, moisturizing and protecting the skin from diaper rash**

(30) Priority: 05.03.1993 US 26883
(71) Applicant: JOHNSON & JOHNSON CONSUMER PRODUCTS, INC., Skillman, New Jersey 08558 (US)
(72) Inventor: Salmon, Michael, Fareham, Hampshire P017 5EL (GB)
(74) Representative: Fisher, Adrian John

(57) **Abstract**

This invention relates to a cleansing cloth or applicator containing a low-viscosity antimicrobial emulsion useful in combatting the microorganisms that cause diaper rash.

## Description

This invention relates to cosmetic applicators comprising absorbent sheets impregnated with an oil-in-water emulsion incorporating various cleansing and moisturizing agents which are particularly adapted to the effective cleansing and moisturizing of skin. These emulsions may also contain antimicrobial agents exhibiting activity against the causative organisms associated with diaper rash.

### Background of the Invention

It has been shown that the most effective way of preventing diaper rash is to cleanse the skin thoroughly and to remove the microorganisms that have been identified as causative. The source of these microorganisms is often the fecal deposits that can remain on a baby's skin while wearing the diaper. Because fecal deposits consist of both water-soluble and oil-soluble matter, however, complete removal of fecal deposits from the diaper area requires both water-based and oil-based cleansing agents. Thus, it is an object of this invention to provide a mechanism for cleansing babies' skin in order both to remove waste deposits and to reduce the number of microorganisms available to cause infection.

A variety of treated cloths which are adapted for skin washing and cleansing are commercially available. Such products are composed of paper or non-woven fabric sheets which are wetted with an aqueous solution of water soluble or water dispersible ingredients. To cleanse skin thoroughly, both water soluble and oil soluble cleansing agents should be incorporated. Incorporating water-insoluble cosmetic ingredients in the form of an oil-in-water emulsion has been described in U.S. Patent 4,559,157 granted Dec. 17, 1985 to Smith et al., "Cosmetic Applicator Useful for Skin Moisturizing".

However, these prior-art emulsions are not very acceptable in that (1) they possess significant viscosity (1300 - 2000 cPs), which makes them difficult to use and which result in the emulsion remaining on the cloth rather than transferring to the baby's skin; or, (2) in cases where viscosity has been reduced, the compositions tend to be unstable. Thus, it has been difficult to make stable compositions that are low in viscosity and therefore acceptable for use on a cleansing cloth or wipe.

Accordingly, it is an object of this invention to provide a cosmetic applicator containing both water-soluble and water-insoluble cleansing agents and an antimicrobial agent, incorporated over and above the requirements for adequately preserving the product, which exhibits activity against the microorganisms that have been shown to be a factor in diaper rash, in a low viscosity, stable form.

It is a further object of the present invention to provide a cosmetic applicator containing ingredients capable of moisturizing skin.

It is a further object of the current invention to provide a cosmetic applicator containing antimicrobial agents possessing activity against the causative micro-organisms associated with diaper rash.

Other objects, advantages and novel features of the this invention will be apparent to those skilled in the art from the following description and appended claims.

### Summary of the Invention

The objects of this invention are attained by providing a cosmetic applicator composed of a sheet of absorbent material which is impregnated with an antimicrobial oil-in-water emulsion. This oil-in-water emulsion contains an oil phase having a least one oil-soluble cleansing agent, at least one oil-soluble moisturizing agent and at least one oil based emulsifying agent dispersed in an aqueous phase. The aqueous phase contains at least one water-soluble detergent, at least one antimicrobial agent exhibiting activity against causative diaper rash micro-organisms and, optionally, some water-soluble emollients or humectants. Effective amounts of antimicrobial preservatives and fragrance may also be employed in the impregnating emulsion.

Such an emulsion would, when applied to the skin via a suitable applicator fabric, provide efficient cleansing of fecal deposits and leave on the skin a residual layer containing an antimicrobial agent capable of acting against the causative micro-organisms associated with diaper rash.

Other objects, advantages and novel features of the present invention will be apparent to those skilled in the art from the following description and appended claims.

### Detailed Description of the Preferred Embodiments

The emulsions of this invention are formulated to be of very low viscosity to enable great flexibility in the choice of impregnation equipment and to enable the ready deposition of the emulsion on the skin.

The emulsions of this invention are formulated so that both the water-soluble and oil-soluble cleansing agents are made available to be deposited upon the skin when the impregnated sheet is pressed or rubbed against a skin surface. Further, the emulsions of this invention have been formulated so that both the oil-soluble and water-soluble moisturizers and emollients and antimicrobial agents are deposited when the impregnated sheet is pressed or rubbed against a skin surface.

In order to create emulsions that are stable during, and subsequent to, manufacture, the emulsions can be formulated by dispersing an oil phase comprising one or more oil-soluble cleansing agents and one or more oil-soluble moisturizing agents and one or more oil-soluble emulsifying agents in an aqueous phase comprising one or more water-soluble cleansing agents and an antimicrobial agent exhibiting activity against causative diaper rash micro-organisms. The aqueous phase may further contain one or more water-soluble moisturizing agents or one or more water-soluble moisturizing agents or humectant. Both the oil phase and the aqueous phase may also incorporate antimicrobial agents in combined amounts effective to prevent bacterial, yeast or fungal growth in the applicators during storage. The emulsions or the present invention may also incorporate an effective amount of fragrance.

Therefore, the emulsions of the present invention preferably will comprise between about 4% and about 50% by weight of active ingredients, i.e. the water-soluble and oil-soluble cleansing agents, emulsifiers, moisturizers, humectant, emollients, antimicrobial agents, fragrance and preservatives; and between about 50 and% about 96% water, preferably distilled or deionized water.

About 3% to about 20% of the active ingredients will be present in the oil phase of the emulsion, while the remainder of the active ingredients will be fully soluble in the aqueous phase.

The emulsions useful in this invention are formulated from an oil phase which incorporates one or more oil-soluble cleansing agents. These cleansing agents are preferably present in an amount of between about 0.5% and about 10% by weight of the entire emulsion.

Oil-soluble cleansing agents function to remove fatty deposits from the skin which are either difficult or impossible to remove using water-soluble detergent cleansers. The oil-soluble cleansing agents useful in the practice of the present invention include those commonly employed in cleansing creams and lotions, such as liquid hydrocarbons such as mineral oil, paraffinic derivatives and the like. In the practice of this invention, iso-paraffins are preferred and may consist of one or more of the commercially available grades such as Isopar-L (Esso Chemical Company U.K.). The iso-paraffin should be present in the amount of about 0.5 to about 10% by weight of the emulsions of the present invention.

The oil phase of the emulsions of the present invention will also include one or more oil-soluble moisturizing agents which are preferably present in an amount between about 1% and about 8% by weight of the entire emulsion. The oil-soluble moisturizing agents useful in the practice of the present invention include those commonly employed in moisturizing creams and lotions such as liquid hydrocarbons such as petrolatum, mineral oil and the like, vegetable and animal fats and oils such as lanolin and its derivatives, vegetable oils and their derivatives), esters and the like. In the practice of the present invention, mineral oils are preferred and may consist of one or more of the commercially available grades such as Carnation White Mineral Oil (Witco Chemical Corporation).

The oil phase of the emulsions of the present invention should also include one or more oil-soluble emulsifying agents. These emulsifying agents should preferably be present in an amount between about 1% and about 7% by weight of the entire emulsion. The oil-soluble emulsifying agents useful in the practice of the present invention include those non-ionic species utilized in the stabilization of cosmetic creams and lotions that contribute little viscosity to the finished formulation e.g. ethoxylated fatty acid-polyol esters, ethoxylated fatty alcohols and fatty esters.

In the practice of this invention, a combination of two or more oil-soluble emulsifying agents is preferably used to improve the stability of the emulsion.

The emulsions of this invention will also preferably include one or more water-soluble emollients or humectant such as polyhydric alcohols, water-soluble lanolin derivatives or the like. In the practice of the present invention polyhydric alcohols such as propylene glycol, glycerin and sorbitol and the like are preferred. The water-soluble emollients or humectant should be present in an amount between about 0.1% and about 8% by weight of the entire emulsion, preferably in the range of between about 0.5% and about 5.0%.

The emulsions of this invention will also include one or more water-soluble detergents or surfactants. These ingredients function both as aqueous water phase cleansing agents and wetting agents for the oil phase ingredients to allow uniform spreading on the skin. The most useful groups of water-soluble detergents are those classified as non-ionic such as ethoxylated sorbitan esters. Detergents that are found not to disturb the stability of the emulsion, including such detergents that are classified as amphoteric, such as imidazoline derivatives, are useful. Those water-soluble detergents or surfactants classed as anionic and cationic may be utilized in the present invention, provided that they do not disturb the stability of the emulsion. However, these anionic and cationic surfactants are not preferred for use in the products of this invention due to their potential to irritate skin.

The water-soluble detergents will preferably be present in an amount between about 1% and about 5% by weight of the entire emulsion.

The emulsions of the present invention will also include one or more bactericidal preservatives in a combined amount to prevent microbial and fungal growth both prior to impregnation and after impregnation. The preservatives useful in the practice of this invention are those commonly used in the formulation of cosmetic creams and lotions (parabens, imidazolidinyl urea, 5-chloro-2-methyl-4-isothiazolin-3-one with 2-methyl-4-isothiazolin-3-one and the like). Preferably the preservatives will make up between about 0.05% and about 1.5% by weight of the entire emulsion.

The emulsions of this invention will include one or more specific antimicrobial agents, incorporated over and above the requirements for adequately preserving the emulsion, which exhibit antimicrobial efficacy against those micro-organisms that have been shown to be a factor in diaper rash e.g., Staphylococcal species, Candida albicans and other reported species. A suitable antimicrobial agent for the purpose of the presentation is Cetyl Pyridinium Chloride. The antimicrobial agents should preferably be present in an amount between about 0.01% and about 1.5% by weight of the entire emulsion.

The emulsions of this present invention may also include an effective amount of fragrance, which may be any of the commercially available perfumes that are compatible with the other ingredients.

The emulsions of the present invention are generally prepared by melting together the oil phase ingredients (oil-soluble cleansers, oil-soluble moisturizers, oil-soluble emulsifiers) with stirring or shaking at temperatures in the range 60 - 85°C. The hot oil phase is then added with agitation to the aqueous phase which has been separately prepared by dissolving the water-soluble emollients or humectant and the water-soluble detergents in water and heating the resultant solution to the same temperature as the oil phase. After a period of stirring or shaking with the temperature maintained at the elevated level, the emulsion is cooled with agitation to 40°C at which point the preservatives, antimicrobial agents and fragrance are added. The emulsion is then cooled further, with agitation, to below 30°C. The finished emulsions should exhibit viscosities of below about 200 cPs, preferably below 20 cPs, and most preferably below 15 cPs, and can therefore be considered to be water-thin. The thinner the emulsion is, the easier it is to impregnate.

The finished emulsions exhibit excellent stability for extended periods of time at all temperatures ranging from 0°C - 50°C.

The finished emulsion is then applied at the desired weight onto one or both sides of an absorbent sheet which may be formed from any woven or non-woven fiber, fiber mixture or foam of sufficient wet strength and absorbency to hold an effective amount of the emulsion.

Cellulosic fibrous webs are preferred as the absorbent sheet for the applicator of the present invention because of their low cost and biodegradability. Especially preferred are paper, air-laid and carded nonwoven webs. However, spun bonded and spun laced webs are also suitable. For applications where cost and/or biodegradability are not important, alveolar polymeric films, foams and other porous sheets may be employed. The types of materials used in such cloths that are particularly useful in the products of this invention are: wood pulp and other cellulosic fibers, viscose, rayon and similar fibers prepared into sheets of between about 25 g/m² and about 100 g/m² by methods well known in the art.

Any method suitable for the application of aqueous or aqueous/ alcoholic impregnants, such as flood coating or metered dosing, can be used to impregnate the fibrous webs with emulsions of this invention. More specialized techniques such as Meyer Rod, floating knife or doctor blade which are more typically sued to impregnate oil-in-water emulsions may also be used but are not necessary.

The emulsion should preferably comprise between about 150 and about 600% based upon the weight of the porous sheet, most preferably between about 200 and about 400%.

After coating, the applicators may be folded into stacks and packaged in any of the moisture and vapor impermeable packages known in the art.

### EXAMPLE I

A cleansing wipe containing a cleansing emulsion according to this invention was made as set forth below. The following ingredients are used in making an emulsion according to this invention.

| Ingredients | Percent |
|---|---|
| Group A (oil phase) | |
| Mineral Oil | 4.00 |
| Isoparaffin | 2.00 |
| PEG-5 Glyceryl Stearate | 1.35 |
| Steareth-10 | 1.35 |

| Group B (aqueous phase) | |
|---|---|
| Deionized Water | 88.25 |
| Polysorbate 20 | 1.00 |
| Propylene Glycol | 1.00 |

| Group C | |
|---|---|
| Kathon CG | 0.10 |
| Imidazolidinyl Urea | 0.20 |
| Benzyl Alcohol | 0.50 |
| Cetyl Pyridinium Chloride | 0.05 |
| Fragrance | 0.20 |

The oil phase ingredients of Group A were mixed and heated to 70°C. The aqueous phase ingredients of Group B were separately mixed and heated to 70°C and then the Group A ingredients were added with agitation. Stirring was continued for 10 minutes, then cooling was commenced. At 40°C the Group C ingredients were added with agitation and the emulsion was cooled to 30°C.

The resultant emulsion was found to have a viscosity of 5 cPs and was applied to a rayon air-laid non-woven fabric at an addition level of 300% based upon the dry fabric weight. The resultant applicators were moist but not over-wet and could readily be used to cleanse a skin surface.

## Claims

1. A cosmetic applicator comprising a porous or absorbent sheet impregnated with an oil-in-water emulsion containing at least one antimicrobial agent in an amount effective to exhibit antimicrobial activity against the microorganisms that have been shown to be a factor in diaper rash.

2. A cosmetic applicator according to claim 1 wherein the emulsion contains both oil-soluble and water-soluble cleansing agents.

3. A cosmetic applicator according to of claim 2 wherein the oil-soluble cleansing agents are selected from the group consisting of liquid hydrocarbons, paraffinic derivatives and mixtures.

4. A cosmetic applicator according to claim 2, wherein said emulsion comprises oil-soluble and water-soluble cleansing agents in an amount between about 0.5 to about 20% by weight.

5. A cosmetic applicator, according to claim 4, wherein said cleansing agents are present in an amount between about 1 and about 10%.

6. A cosmetic applicator, according to claim 2 wherein the water-soluble cleansing agents are selected from the group consisting of non-ionic, amphoteric and anionic surfactants.

7. A cosmetic applicator according to claim 6 wherein said emulsion comprises said surfactants in an amount between about 0.5% and about 10% by weight.

8. A cosmetic applicator according to claim 7 wherein said emulsion comprises said surfactants in an amount between about 1% and about 5%.

9. A cosmetic applicator according to claim 1 wherein the emulsion further comprises at least one oil-soluble moisturizing agent in an amount between about 0.5% and about 15%.

10. A cosmetic applicator according to claim 9 wherein said oil-soluble moisturizing agent is in an amount between about 1% and about 8%.
